# EUROPEAN PATENT SPECIFICATION

(11) **EP 2 271 929 B1**
(45) Date of publication and mention of the grant of the patent: **25.11.2015**
(21) Application number: 09732031.1
(22) Date of filing: 17.04.2009
(51) Int. Cl.: G01N 31/00, G01N 33/03, G01N 33/92, A61P 3/04, A61P 3/06, A61P 3/10

(54) **USE OF NITRATED LIPIDS FOR TREATMENT OF LIPID DISORDERS AND OBESITY, AND LIPID- AND OBESITY-RELATED CONDITIONS**
VERWENDUNG NITRIERTER LIPIDE ZUR BEHANDLUNG VON LIPIDSTÖRUNGEN UND FETTLEIBIGKEIT SOWIE LEIDEN IN VERBINDUNG MIT LIPIDEN UND FETTLEIBIGKEIT
UTILISATION DE LIPIDES NITRÉS POUR LE TRAITEMENT DE TROUBLES LIPIDIQUES ET DE L'OBÉSITÉ, ET D'AFFECTIONS LIPIDIQUES ET LIÉES À L'OBÉSITÉ

(30) Priority: 18.04.2008 US 46008
(43) Date of publication of application: 12.01.2011
(73) Proprietor: The University of Utah Research Foundation, Salt Lake City, Utah 84108 (US)
(72) Inventor: YANG, Tianxin, Salt Lake City UT 84124 (US)
(74) Representative: Potter Clarkson LLP
(86) International application number: PCT/US2009/041018
(87) International publication number: WO 2009/129495

(56) References cited:
- US-A1- 2007 232 579
- US-A1- 2007 275 893
- US-B2- 6 998 395
- BONACCI GUSTAVO ROBERTO ET AL: "Nitro-Oleic Acid Improves Insulin Signaling Via Protein Tyrosine Phosphatase-1b Inhibition", FREE RADICAL BIOLOGY AND MEDICINE, ELSEVIER SCIENCE, US, vol. 45, no. Suppl. 1, 1 January 2008 (2008-01-01), page S154, XP009152935, ISSN: 0891-5849
- LIMA EMERSOM S ET AL: "Cholesteryl nitrolinoleate, a nitrated lipid present in human blood plasma and lipoproteins", JOURNAL OF LIPID RESEARCH, AMERICAN SOCIETY FOR BIOCHEMISTRY AND MOLECULAR BIOLOGY, INC, US, vol. 44, no. 9, 1 September 2003 (2003-09-01), pages 1660-1666, XP009152938, ISSN: 0022-2275
- DEL MAR GRASA M ET AL: "Daily oral oleoyl-estrone gavage induces a dose-dependent loss of fat in Wistar rats", OBESITY RESEARCH, BATON ROUGE, LA, US, vol. 9, no. 3, 1 March 2001 (2001-03-01), pages 202-209, XP002520845, ISSN: 1071-7323, DOI: 10.1038/OBY.2001.22
- LIMA E S ET AL: "Nitrated lipids decompose to nitric oxide and lipid radicals and cause vasorelaxation", FREE RADICAL BIOLOGY AND MEDICINE, ELSEVIER SCIENCE, US, vol. 39, no. 4, 15 August 2005 (2005-08-15), pages 532-539, XP004989020, ISSN: 0891-5849, DOI: 10.1016/J.FREERADBIOMED.2005.04.005

## Description

### FIELD OF THE INVENTION

The present invention relates to nitrated lipids for use in methods of treating lipid disorders, obesity and obesity-related conditions and disorders using nitrated lipids. In particular, the methods comprise the use of nitrated fatty acids or esters thereof to reduce the level of triglycerides and free fatty acids and/or to reduce the body weight of a subject and/or to increase the level of high-density lipoprotein (HDL), and/or reduce oxidative stress in a subject.

### BACKGROUND

The prevalence of overweight and obese persons has dramatically increased during the past two decades. Currently, 65% of adults in the United States are overweight and 31% of adults are obese. The incidence of obesity in children has also risen dramatically. Obesity, along with hypertriglyceridemia, represents a major risk factor for type 2 diabetes, hypertension, and other chronic diseases. As a result, 18 million Americans are living with diabetes and every minute another American is diagnosed with this disease.

Modulators of peroxisome proliferation-activated receptors (PPAR) have proven useful in treating certain aspects of lipid and obesity-related conditions and disorders. Thiazolidinediones (TZDs), *e*.*g*., rosiglitazone and pioglitazone, are synthetic PPARγ activators which are highly effective for the control of hyperglycemia but have limited beneficial effects on lipid profiles. TZDs are also associated with several major side effects such as edema, body weight gains, and an increased incidence of chronic heart failure. These side effects often require discontinuation of therapy. Clofibrate and fenofibrate, synthetic PPARa activators, are clinically used as lipid lowering agents but have limited potency. Recently developed dual PPARα/γ agonists have the goal of achieving similar control of both hyperglycemia and hypertriglyceridemia. However, muraglitazar, the first dual PPARα/γ agonist, is associated with an excess incidence of the composite end point of death, major adverse cardiovascular events (myocardial infarction, stroke and transient ischemic attack), and chronic heart failure.

An article by Bonacci et al. entitled "Nitro-Oleic Improves Insulin Signalling Via Protein Tyrosine Phosphatase-1b Inhibition" was published in Free Radical Biology and Medicine 45(Suppl. 1), S154 (1 January 2008).

An article by Lima et al. entitled "Cholesteryl nitrolinoleate, a nitrated lipid present in human blood plasma and lipoproteins" was published in J. Lipid Res. 44(9), 1660-1666 (1 September 2003).

An article by del Mar Grasa et al. entitled "Daily oral oleyl-estrone gavage induces a dose-dependent loss of fat in Wistar rats" was published in Obesity Res. 9(3), 202-209 (1 March 2001).

An article by Lima et al. entitled "Nitrated lipids decompose to nitric oxide and lipid radicals and cause vasorelaxation" was published in Free Radical Biology and Medicine 39(4), 532-539 (15 August 2005).

US 2007/232579, entitled "Nitrated Lipids and Methods of Making and Using Thereof" was published on 4 October 2007.

### SUMMARY

The present technology provides nitrated lipids for use in methods useful in the treatment of lipid disorders, obesity, and lipid- and obesity-related conditions or disorders. The methods include administration of at least one nitrated lipid to a subject in need thereof in amounts effective to treat one or more of lipid disorders, obesity, and lipid- and obesity-related conditions or disorders. Nitrated lipids provide a number of advantages over existing compounds and therapies for the treatment of lipid disorders, obesity, and lipid- and obesity-related conditions. First, nitrated lipids can exhibit superior efficacy and safety profiles over synthetic compounds. For example, nitrated oleic acid (OA-NO₂) can achieve a similar efficacy at much lower dosages (at least 100 times less) than rosiglitazone. Second, nitrated lipids exhibit robust lipid lowering effects not typically seen with synthetic compounds. Third, in surprising and unexpected contrast to TZDs, which are associated with body weight gains, nitrated lipids induce body weight loss.

The nitrated lipids are for use in methods that comprise administering an effective amount of a nitrated lipid to a subject in need thereof. A variety of lipids may be used to form the nitrated lipids, including fatty acids or esters thereof. Similarly, a variety of fatty acids are compatible with the disclosed methods, including monounsaturated and polyunsaturated fatty acids. Procedures for synthesizing nitrated lipids, sources for obtaining the lipids, and administration routes for the nitrated lipids are also provided herein.

The nitrated lipids are to be administered in an amount effective to treat one or more of lipid disorders, obesity, lipid-related conditions or disorders and obesity-related conditions or disorders. The lipid disorder is one or more of hypertriglyceridemia, high free fatty acid concentration, and low high-density-lipoprotein-cholesterol concentration (hypo-HDL-C). The lipid disorder may or may not be associated with obesity. The lipid-related conditions or disorders and obesity-related conditions or disorders is one or more of obesity- or lipid-related organ injuries or dysfunction, proteinuria, retinopathy, neuropathy, and nephropathy.

The effective amount of the nitrated lipid administered to the subject may vary. In some aspects, the effective amount is that which reduces the body weight of a subject or slows or halts the subject's body weight gain relative to the subject's body weight or body weight gain prior to administration of the nitrated lipid. In other aspects, the effective amount is that which reduces one or more of the subject's food intake, triglyceride level, free fatty acid level, glucose level, cholesterol level, insulin level, oxidative stress level and/or rate of urinary protein excretion. In other aspects, the effective amount is that which increases HDL levels in a subject. The levels or rates are increased or reduced in a subject relative to the subject's level or rate prior to the administration of the nitrated lipid. In yet further aspects, the effective amount is that which provides various combinations of these treatment results.

The nitrated lipids are for use in methods disclosed herein that may further comprise administrating a variety of therapeutic agents useful in the treatment of lipid disorders, lipid-related conditions or disorders, obesity or obesity-related conditions or disorders. In addition, the methods encompass administering the nitrated lipid in combination with other therapies.

### BRIEF DESCRIPTION OF THE FIGURES

**Figure 1****.** Body weight gain in male 4-mo-old Zucker obese rats (obese) after 14-d infusion with vehicle, OA-NO₂, and OA, each at 7.5 µg/kg/d via osmotic mini-pumps. Age and gender matched lean rats with vehicle treatment were used as controls. Lean: n=3; Vehicle: n=7; OA-NO₂: N=5; OA: n=5. *, *P*<0.05 vs. Obese/Vehicle. Data are mean ± Standard Error (SE).
**Figure 2****.** Food intake was determined in the Zucker obese rat at the indicated time points. *, *P*<0.05 vs. Obese/Vehicle at the corresponding time period. Lean: n=3; Vehicle: n=7; OA-NO₂: N=5; OA: n=5. Data are mean ± SE.
**Figure 3****.** Effects of OA-NO₂ on plasma triglyceride (A) and plasma non-esterified fatty acid (B) in Zucker obese rats. The assays were performed before and 14 days after treatment. Lean: n=3; Vehicle: n=7; OA-NO₂: N=5; OA: n=5. (A) *, *P*<0.05 vs. Obese/Vehicle; (B) #, P<0.001 vs. Obese/Vehicle or Obese/Day 0. Data are mean ± SE.
**Figure 4****.** Effects of OA-NO₂ versus OA on hypercholesterolemia in Zucker obese rats. Plasma cholesterol was determined before and 14 days after treatment. Lean: n=3; Vehicle: n=7; OA-NO₂: N=5; OA: n=5. Data are mean ± SE.
**Figure 5****.** Effects of OA-NO₂ versus OA on plasma HDL in Zucker obese rats. Plasma HDL (A) and the ratio of HDL to total cholesterol (B) were determined before and 14 days after treatment. Lean: n=3; Vehicle: n=7; OA-NO₂: N=5; OA: n=5. *, *P*<0.05 vs Day 0 in the same group or vs. Obese/Vehicle. Data are mean ± SE.
**Figure 6****.** Effects of OA-NO₂ versus OA on plasma TBARS in Zucker obese rats. *, *P*<0.05 vs. Day 0 in the same group; #, *P*<0.01 vs. Obese/Vehicle. Lean: n=3; Vehicle: n=7; OA-NO₂: N=5; OA: n=5. Data are mean ± SE.
**Figure 7****.** Effects of OA-NO₂ versus OA on proteinuria in Zucker obese rats. *, *P*<0.05 vs. Day 0 in the same group; #, *P*<0.01 vs. Obese/Vehicle. Lean: n=3; Vehicle: n=7; OA-NO₂: N=5; OA: n=5. Data are mean ± SE.
**Figure 8****.** Effects of OA-NO₂ on hyperglycemia (A) and hyperinsulinemia (B) in the Zucker diabetic rat (ZDF strain). ZDF rats were infused for 1 week with OA-NO2 at 7.5 µg/kg/d via osmotic mini-pumps. Plasma glucose and insulin were determined before and after OA-NO₂ treatment. The blood sampling was performed after fasting from 6:00PM to 9:00AM. N=4 per group. *, *P*<0.05 vs. Day 0. Data are mean ± SE.

### DETAILED DESCRIPTION

The present invention relates to nitrated lipids for use in treating one or more lipid disorders, obesity, lipid-related conditions or disorders, or obesity-related conditions or disorders according to the attached claims.

The following terms are used throughout as defined below.

"Obesity" is generally defined as a body mass index (BMI) over 30. "Morbid obesity" refers to a BMI of 40 or greater. However, for purposes of this disclosure, subjects with a BMI of 25-30 (considered overweight), or 25 and above are also included within the scope of "obese." However, subjects at or below a BMI of 25 may also be subjects in the present methods of treatment.

"Obesity-related conditions or disorders"; "Lipid disorders" and "Lipid-related conditions and disorders" are as defined in the attached claims.

"Treating" means an alleviation, in whole or in part, of symptoms associated with a condition or disorder (*e*.*g*., disease), or halt of further progression or worsening of those symptoms, or prevention or prophylaxis of the disease or disorder. Similarly, as used herein, an "effective amount" of a compound disclosed herein refers to an amount of the compound that alleviates, in whole or in part, symptoms associated with a condition or disorder, or halts further progression or worsening of those symptoms, or prevents or provides prophylaxis for the disease or disorder. For example, in treating obesity, a slowed or halted increase in body weight or a decrease in body weight are examples of desirable treatment results. In yet another example, treating obesity may include the prevention of obesity in a child. The child may be susceptible to, or genetically predisposed to, obesity or obesity-related conditions. Similarly, an effective amount of a compound disclosed herein may refer to an amount of the compound that prevents obesity in a child. As another example, an effective amount of a compound for treatment of a lipid-related disorder or condition such as hypertriglyceridemia, may slow, halt or provide a decrease in the level of triglycerides in the child or other subject. Further, treating does not necessarily occur by administration of one dose of the compound, but often occurs upon administration of a series of doses. Thus, an effective amount, an amount sufficient to alleviate, or an amount sufficient to treat a disease, disorder, or condition may be administered in one or more doses or a single dose spread over one or more administrations.

The nitrated lipids may be for use in methods disclosed herein that comprise administering an effective amount of a nitrated lipid to a subject in need thereof. Nitrated lipids are lipids comprising at least one nitro (NO₂) group covalently bonded to the lipid. The nitrated lipids may be for use in methods disclosed herein that encompass administration of a single type of nitrated lipid or a mixture of two or more different types of nitrated lipids. By way of example, a single type of nitrated lipid is 9-nitro-9-*cis-*octadecanoic acid. Thus, "type" identifies the compound by lipid, stereochemistry, and number and position of NO₂ groups.

Nitrated lipids include nitrated fatty acids or esters thereof. A fatty acid is a substituted or unsubstituted alkyl or alkenyl having a terminal COOH group. In some embodiments, the alkyl or alkenyl is a C₈-C₂₄ alkyl or alkenyl. A wide variety of fatty acids may be used, including monounsaturated fatty acids and polyunsaturated fatty acids. In some embodiments, the monounsaturated fatty acid is oleic acid. In some embodiments, the oleic acid is 9-nitrooleic acid, 10-nitrooleic acid, or combinations thereof. An ester of a fatty acid is a substituted or unsubstituted alkyl or alkenyl having a terminal COOR group. In some embodiments, the alkyl or alkenyl is a C₈-C₂₄ alkyl or alkenyl. R may include a C₁₋₈ alkyl or glyceryl.

Nitrated lipids may be synthesized according to known procedures. For example, U.S. Patent Publication No. 2007/0232579 discloses a procedure comprising the steps of reacting a lipid with a mercuric salt, a selenium compound, and a nitrating compound to produce a first intermediate and reacting the first intermediate with an oxidant. Useful mercuric salts, selenium compounds, nitrating compounds, oxidants, relative amounts of reactants, and reaction conditions are also disclosed in U.S. Patent Publication No. 2007/0232579. Such synthetic procedures may provide mixtures of two or more types of nitrated lipids which may be separated or purified by techniques known in the art, if desired.

The lipids described above may be obtained from a variety of sources. For example, lipids may be commercially available or may be obtained from natural sources. Plant oils, including olive oil, linseed oil, flaxseed oil, rapeseed oil, and perilla oil are possible natural sources of fatty acid lipids. Fish oils or other marine oils are other possible sources of fatty acids. Nitrated lipids present in any of these or other natural sources may be extracted and/or purified for use in the methods disclosed herein.

The nitrated lipids are to be administered in an amount effective to treat one or more conditions selected from lipid disorders, obesity, lipid-related conditions or disorders and obesity-related conditions or disorders. The lipid disorder is one or more of hypertriglyceridemia, high free fatty acid concentration, and low high-density-lipoprotein-cholesterol concentration (hypo-HDL-C). The lipid disorder may or may not be associated with obesity. The lipid-related conditions or disorders and obesity-related conditions or disorders is one or more of obesity- or lipid-related organ injuries or dysfunction, including proteinuria, retinopathy, neuropathy, and nephropathy.

The nitrated lipids are to be administered to a subject in an effective amount. In some embodiments, the effective amount is an amount that reduces the body weight of the subject relative to the subject's body weight prior to administration of the nitrated lipid. In some embodiments, the subject's body weight is reduced from by about 1% to by about 20%, by about 5% to by about 20%, or by about 5% to by about 15%. In other embodiments, the effective amount is that which slows or halts the subject's body weight gain relative to the subject's body weight gain prior to administration of the nitrated lipid. In other embodiments, the effective amount is that which reduces the subject's body food intake relative to the subject's food intake prior to administration of the nitrated lipid. In other embodiments, the subject's food intake is reduced from by about 10% to by about 70%, by about 15% to by about 50%, or by about 20% to by about 30%.

In further embodiments, the nitrated lipids are for use in methods that involve treatment of a subject suffering from a lipid disorder or a lipid-related disorder or condition, e.g., a subject in need of a reduction in the subject's triglyceride, cholesterol, free fatty acid, urine protein levels, or oxidative stress levels, or an increase in a subject's plasma high-density lipoprotein (HDL) level. Thus, in some embodiments, the amount is effective to reduce the level of triglycerides in the subject, relative to the subject's level prior to administration of the nitrated lipid. In some such embodiments, the subject's triglyceride level is reduced by from about 5% or about 10% to about 50%, from about 15% to from about 45%, or from about 20% to about 40%. In further embodiments, the amount is effective to reduce the level of plasma free fatty acid in the subject, relative to the subject's level prior to administration of the nitrated lipid. In some such embodiments, the subject's plasma free fatty acid level is reduced by from about 10% to about 90%, from about 20% to about 70%, or from about 30% to about 60%. In other embodiments, the amount is effective to decrease the level of urine protein. In some such embodiments, the subject's plasma urine protein is reduced by from about 10% to about 90%, from about 20% to about 70%, or from about 30% to about 60%. In further embodiments, the amount is effective to increase the level of plasma HDL in the subject, relative to the subject's level prior to administration of the nitrated lipid. In some such embodiments, the subject's plasma HDL level is increased by from about 10% to about 90%, from about 15% to about 70%, or from about 20% to about 50%.

In further aspects, the methods involve a reduction in a subject's glucose level, and/or insulin level. Thus, in some embodiments, the amount is effective to reduce the level of glucose in the subject, relative to the subject's level prior to administration of the nitrated lipid. In such embodiments, the subject's glucose level is reduced by from about 10% to about 60%, from about 15% to about 50%, or from about 20% to about 45%. In further embodiments, the amount is effective to reduce the level of cholesterol in the subject, relative to the subject's level prior to administration of the nitrated lipid. In such embodiments, the subject's cholesterol level is reduced by from about 0% to about 60%, from about 10% to about 50%, or from about 20% to about 40%. In other embodiments, the amount is effective to reduce the level of insulin in the subject, relative to the subject's level prior to administration of the nitrated lipid. In such embodiments, the subject's insulin level is reduced by from about 10 % to about 90%, from about 20% to about 80%, or from about 20% to about 50%.

In some embodiments, the methods involve a reduction in a subject's oxidative stress level. Oxidative stress is reported to play a pathogenic role in the development of metabolic syndrome. Oxidative stress may or may not be associated with obesity or hyperlipidemia. Thus, in some embodiments, the amount is effective to reduce the level of oxidative stress in the subject, relative to the subject's level prior to administration of the nitrated lipid. In such embodiments, plasma Thiobarbituric acid (TBARS), is measured an index of systemic oxidative stress. Thus, in some embodiments, the amount is effective to reduce the level of plasma TBARS in the subject, relative to the subject's level prior to administration of the nitrated lipid. In some such embodiments, the subject's plasma TBARS level is reduced by from about 10% to about 90%, from about 20% to about 70%, or from about 30% to about 60%.

The methods disclosed herein further encompass administering a nitrated lipid in amounts effective to provide various combinations of the treatment results described above. For example, in some embodiments, the effective amount of the nitrated lipid is that which reduces both the subject's body weight and triglyceride level. In other embodiments, the effective amount is that which reduces the subject's triglyceride level and/or one or more of the subject's glucose level, insulin level and rate of urine protein excretion. In still further embodiments, the effective amount is that which reduces the subject's body weight, triglyceride level and/or one or more of the subject's glucose level, insulin level and rate of urine protein excretion. In other embodiments, the effective amount is that which reduces the subject's body weight, triglyceride level and increases the subject's plasma HDL level. In yet another embodiments, the effective amount is that which reduces the subject's body weight, triglyceride level and urine protein level.

Specific effective amounts of the nitrated lipids to be administered will vary depending upon a variety of factors, *e*.*g*., the condition to be treated, the age, body weight, general health, sex, and diet of the subject, the dose intervals, and the administration route. In some embodiments, the effective amount of the nitrated lipid ranges from about 1 µg per day to about 1 g per day, from about 1 mg per day to about 500 mg per day, from about 1 mg per day to about 100 mg per day, or from about 2 mg per day to about 10 mg per day.

Any of the nitrated lipids disclosed herein may be for use in methods in which they are administered to the subject alone or in combination with one or more other therapeutic agents. By "administered in combination," it is meant that the nitrated lipids and the therapeutic agents may be administered as a single composition, simultaneously as separate doses, or sequentially. Sequential administration refers to administering the nitrated lipids and at least one therapeutic agent either before or after one another. A variety of therapeutic agents may be used, including those useful in the treatment of a lipid disorders, lipid-related disorders or conditions, obesity or any of the obesity-related conditions or disorders described above. A nitrated lipid can be co-administered with other therapies which include surgery with sleeve gastrectomy, nonpharmacological therapy with life-style changes, pharmacological therapies with statin, fibrate, nicotinic acid, and omega-3 fatty acid or fish oil.

The nitrated lipids may be for use in methods in which they are administered to a subject via any number of pharmaceutical formulations and administration routes. The formulations can take the form of granules, powders, tablets, capsules, syrup, suppositories, injections, emulsions, elixirs, suspensions or solutions. These formulations may further include a variety of well-known pharmaceutically acceptable additives, carriers, and/or excipients as necessary. The formulations may be delivered to the subject by various routes of administration, *e*.*g*., by topical administration, transdermal administration, oral administration, by nasal administration, rectal administration, subcutaneous injection, intravenous injection, intramuscular injection, or intraperitoneal injection. Any of the formulations, delivery methods, and pharmaceutically acceptable additives, carriers, and excipients disclosed in U.S. Patent Publication No. 2007/0232579 may also be used with the methods described herein. Another possible route of administration includes incorporating the nitrated lipid into various food products. Food products, include, but are not limited to butter, margarine, vegetable oils, and the like.

The subjects of the disclosed methods include any animal that can benefit from the administration of a nitrated lipid. In some embodiments, the subject is a mammal, *e*.*g*., a human, a primate, a dog, a cat, a horse, a cow, a pig, or a rodent, *e*.*g*., a rat or mouse. Typically, the mammal is a human. The human may be an adult or a child. The human may be susceptible to, or genetically predisposed to, a lipid-related disorder or condition, obesity or obesity-related disorders or conditions.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a nonlimiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into subranges as discussed above. Finally, as will be understood by one skilled in the art, a range includes each individual member. Thus, for example, a group having 1-3 atoms refers to groups having 1, 2, or 3 atoms. Similarly, a group having 1-5 atoms refers to groups having 1,2,3, 4, or 5 atoms, and so forth.

For the purposes of this disclosure and unless otherwise specified, "a" or "an" means "one or more."

The present technology, thus generally described, will be understood more readily by reference to the following examples, which are provided by way of illustration and are not intended to be limiting of the present technology.

### EXAMPLES

### Materials and Methods:

### Animals

Male 4-month-old Zucker obese and Zucker diabetic rats were from Charles River Laboratory (Wilmington, MA). All animals were housed in an air-conditioned room with a 12-hour light/dark cycle. All procedures and protocols were in accordance with guidelines set by the Laboratory Animal Care Committee at the University of Utah.

### Materials

9-Nitrooleic acid and 10-nitrooleic acid are two regioisomers of nitrooleic acid (OA-NO₂), which are formed by nitration of oleic acid in approximately equal proportions *in vivo*. *See* Baker P.R. et al., Fatty acid transduction of nitric oxide signaling: multiple nitrated unsaturated fatty acid derivatives exist in human blood and urine and serve as endogenous peroxisome proliferator-activated receptor ligands, J Biol Chem, 280: 42464-42475,2005. The two compounds were purchased from Cayman Chemicals (Ann Arbor, MI) (9-nitrooleic acid: Cat#10008042; 10-nitrooleic acid: Cat#10008043) and used as an 1:1 mixture of the isomers.

Oleic acid was purchased from Sigma (Cat#75090).

### Protocols for animal experiments.

### Protocol I for chronic infusion of OA-NO₂.

Two strains of Zucker rats, obese and diabetes (ZDF) (Charles River), were treated with vehicle (ethanol), OA-NO₂ at 7.5 µg/kg/d, or OA at 7.5 µg/kg/d via an osmotic mini-pump. Under isoflurane anesthesia, the mini-pump was implanted under the skin of the neck area. Body weight, food intake, and collections of blood and urine samples were determined before and after treatment. Animals were fasted from 6:00 pm to 9:00 am before blood sampling. Blood sampling was conducted by making a small cut (~2 mm) in the tail using razor blade. 24-hour urine was collected using metabolic cages.

### Example 1: The Use of Nitrated Lipids for Treatment of Obesity and Hyperlipidemia and Kidney Injury in Zucker Obese Rats.

Adult Zucker obese rats develop severe obesity and hyperlipidemia but are relatively resistant to diabetes. Therefore, these animals were used for testing the weight-loss and lipid lowering effects of OA-NO₂. To determine whether these effects are directly attributable to the nitration of the fatty acid, the parent fatty acid (OA) was used as a control. Starting from 4 mo of age, Zucker obese rats were chronically infused for 2 weeks with vehicle, OA-NO₂ (9-nitrooleic acid and 10- nitrooleic acid are purchased from Cayman Chemical, Ann Arbor, MI and used as an 1:1 mixture), or OA each at 7.5 µg/kg/day via an osmotic mini-pump. Parameters including body weight, food intake, the lipid profile, oxidative stress, and urinary protein excretion were determined. Animals were fasted from 6:00 pm to 9:00 am before blood sampling. The following results were observed.

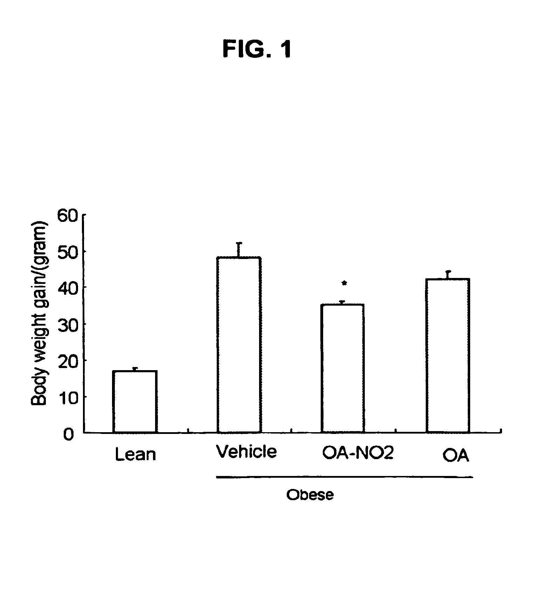

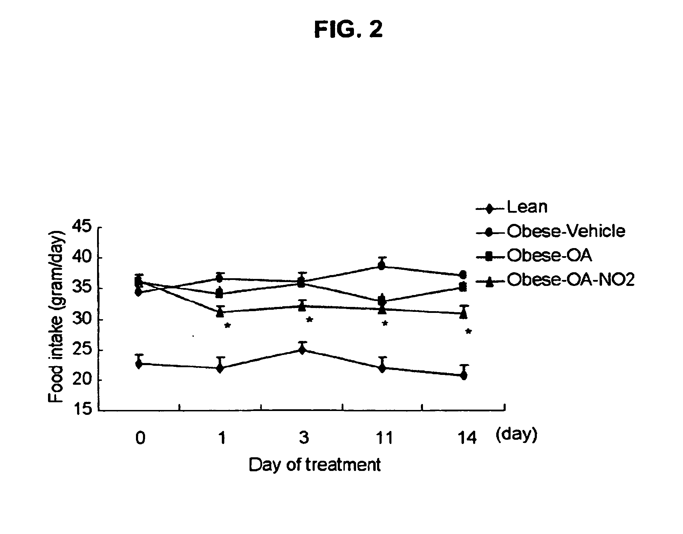

After 2 weeks of treatment, the body weight gain in the OA-NO₂ group was less than that in the vehicle control (Figure 1). In contrast, the value was not different between the OA group and the vehicle control. In parallel with the changes in body weight gain, food intake was significantly reduced by OA-NO₂ but not OA (Figure 2). The reduction of food intake likely explains the body weight loss. The findings suggest that OA-NO₂ may suppress appetite.

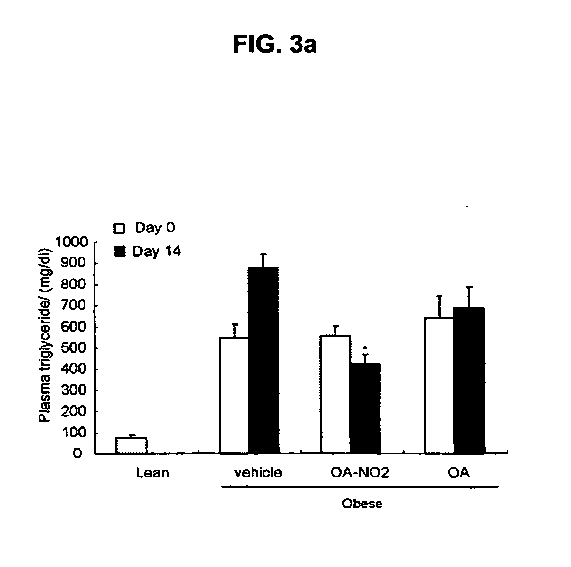

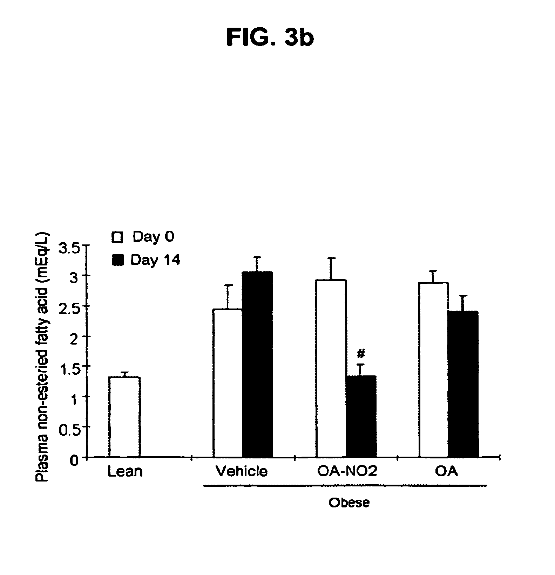

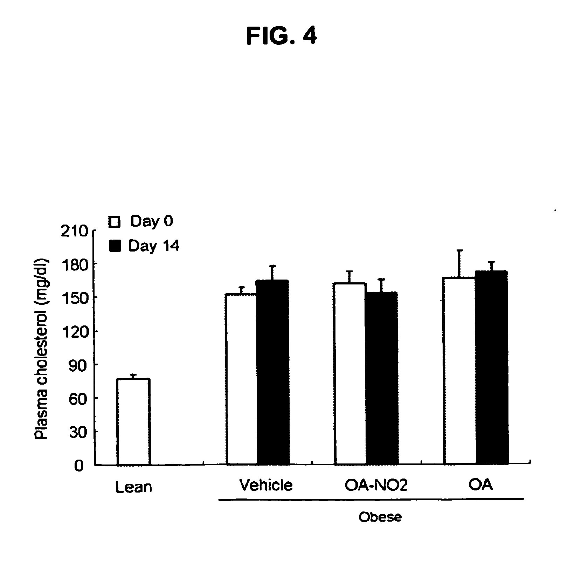

A 2 week administration of OA-NO₂, but not OA, produced a significant triglyceride-lowering effect as compared with vehicle control (Figure 3A). Strikingly, OA-NO₂ almost completely normalized plasma free fatty acid levels (Figure 3B) contrasting to the lack of an effect of OA. While, plasma cholesterol levels were unaffected by OA-NO₂ or OA (Figure 4).

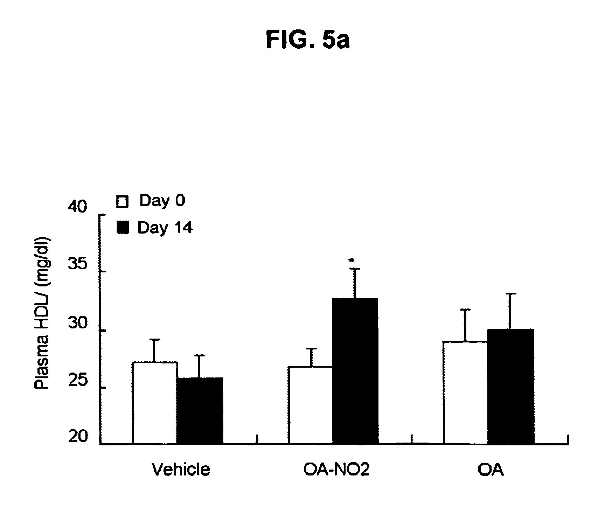

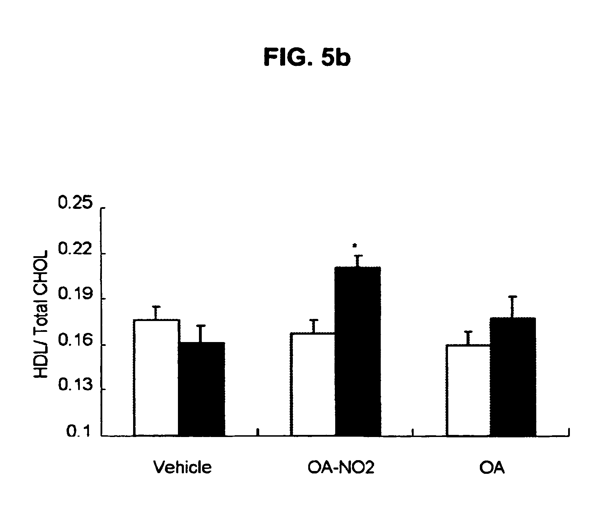

HDL cholesterol is known as "good" cholesterol, because high levels of HDL are suggested to protect against heart attack. We found that OA-NO₂ treatment significantly increased plasma HDL levels but OA was ineffective (Figure 5A). Similar patterns were seen when the data was expressed as the ratio of HDL to total cholesterol (Figure 5B). Therefore, OA-NO₂ appeared to exert dual beneficial effects on dyslipidemia by decreasing "bad" lipids and increasing "good" lipids.

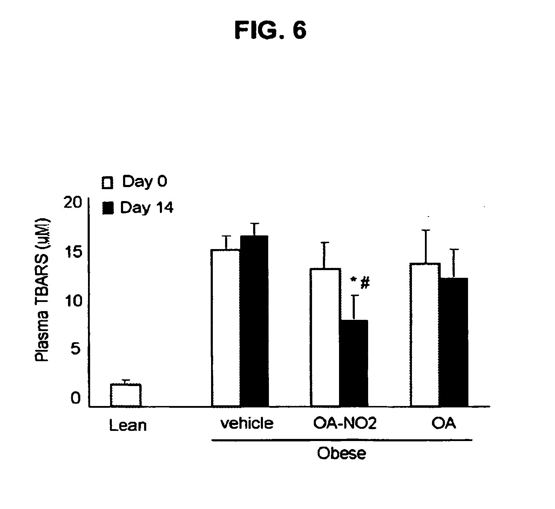

Oxidative stress is reported to play a pathogenic role in the development of metabolic syndrome. We examined the possibility that OA-NO₂ may act by reducing oxidative stress. Plasma TBARS, an index of systemic oxidative stress, markedly increased in obese Zucker rats as compared with lean controls. This increase was significantly attenuated by OA-NO₂ but not OA (Figure 6).

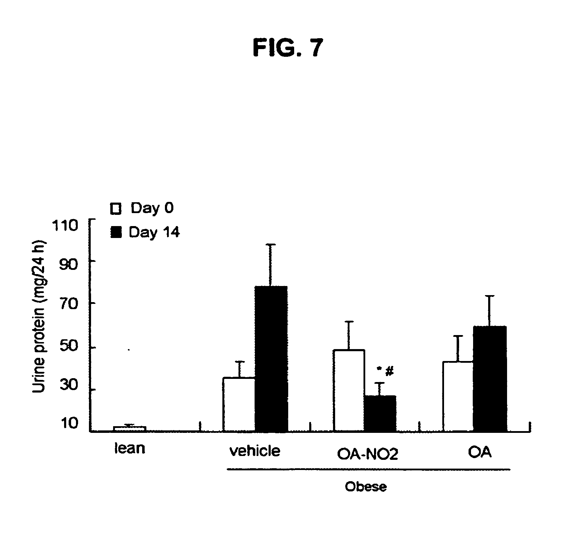

Proteinuira is an index of kidney injury and is viewed as an important component of metabolic syndrome. We examined effect of OA-NO₂ versus OA on proteinuria. Obese Zucker rats developed marked proteinuria as compared with lean controls. A 2 week OA-NO₂ treatment reduced proteiuria by 50% and OA treatment had no significant effect (Figure 7).

### Example 2: The Use of Nitrated Lipids for the Treatment of Diabetes in Zucker Diabetic Rats.

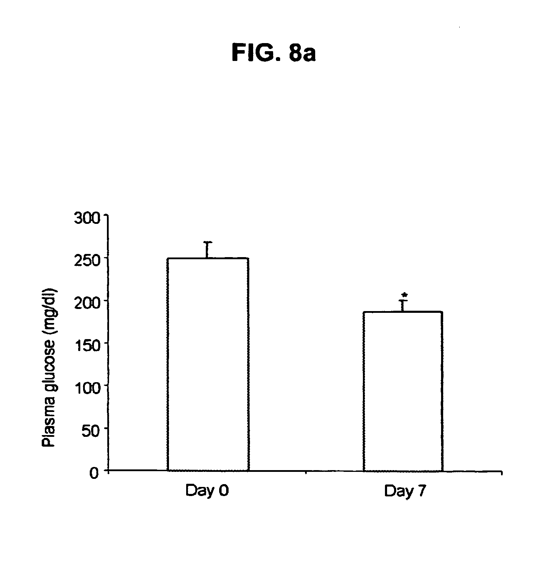

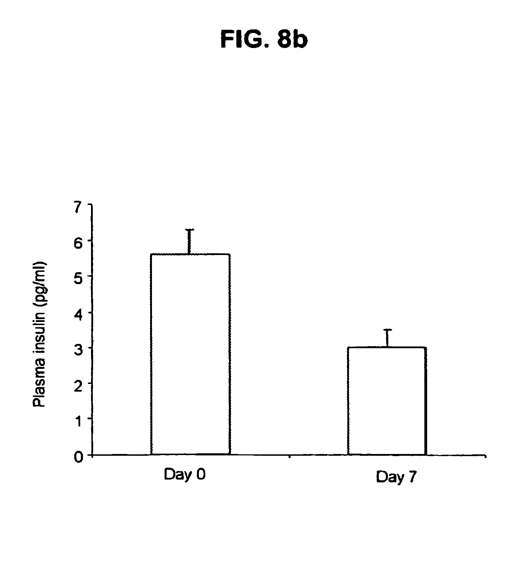

To evaluate the efficacy of OA-NO₂ for treatment of diabetes, Zucker diabetic rats were used. These animals develop severe hyperglycemia and hyperinsulinemia, the two major parameters of type 2 diabetes. We found that administration of OA-NO₂ at 7.5 µg/kg/d via osmotic mini-pumps significantly attenuated hyperglycemia in Zucker diabetic rats (Figure 8A). This treatment tended to reduce plasma insulin levels but this effect did not reach a statistical significance (Figure 8B).

## Claims

1. A nitrated lipid for use in treating one or more of lipid disorders, obesity, lipid-related conditions or disorders, or obesity-related conditions or disorders, wherein the lipid disorder is selected from one or more of hypertriglyceridemia, high free fatty acid concentration, and low high-density-lipoprotein-cholesterol concentration (hypo-HDL-C) and optionally wherein the lipid disorder is associated with obesity and the lipid-related conditions or disorders and obesity-related conditions or disorders are selected from lipid- or obesity-related organ injuries or dysfunction, proteinuria, retinopathy, neuropathy, or nephropathy.

2. The nitrated lipid for use according to claim 1, wherein the level of one or more of triglycerides, free fatty acids, body weight, cholesterol, urine protein excretion and oxidative stress is reduced or the level of plasma HDL is increased in a subject relative to the subject's level or rate prior to treatment with the nitrated lipid.

3. The nitrated lipid for use according to claim 1, wherein the nitrated lipid is administered to a subject who is also administered one or more therapeutic agents, wherein the therapeutic agent is selected from agents useful in the treatment of the lipid disorders defined in claim 1, obesity or the lipid-related conditions or disorders or obesity-related conditions or disorders defined in claim 1.

4. The nitrated lipid for use according to claim 1, wherein the nitrated lipid comprises a monounsaturated fatty acid or a polyunsaturated fatty acid.

5. The nitrated lipid for use according to claim 4, wherein the monounsaturated fatty acid is oleic acid.

6. The nitrated lipid for use according to claim 5, wherein the monounsaturated fatty acid is selected from 9-nitrooleic acid, 10-nitrooleic acid, or combinations thereof.

7. The nitrated lipid for use according to claim 1, wherein the nitrated lipid is administered in an amount ranging from about 1 µg to 1 g per day.

8. The nitrated lipid for use according to claim 2, wherein the body weight of the subject is reduced, relative to the subject's body weight prior to treatment with the nitrated lipid.

9. The nitrated lipid for use according to claim 8, wherein the reduction is from 5% to 15%.

10. The nitrated lipid for use according to claim 2 wherein the level of triglycerides in the subject is reduced, relative to the subject's level prior to treatment with the nitrated lipid.

11. The nitrated lipid for use according to claim 10, wherein the reduction is from 10% to 50%.

12. The nitrated lipid for use according to claim 2 wherein the level of free fatty acid in the subject is reduced, relative to the subject's level prior to the treatment with the nitrated lipid, and wherein the reduction is optionally from 10% to 90%.

13. The nitrated lipid for use according to claim 2 wherein the level of urine protein excretion in the subject is reduced, relative to the subject's level prior to the treatment with the nitrated lipid, and optionally wherein the rate of urine protein excretion is reduced from 20% to 70%.

14. A nitrated lipid for use according to claim 2 wherein the level of plasma HDL in the subject is increased, relative to the subject's level prior to the treatment with the nitrated lipid, and optionally wherein the level of plasma HDL is increased from 10% to 90%.

15. The nitrated lipid for use according to claim 1, wherein the subject is obese or morbidly obese.

## Patentansprüche

1. Nitriertes Lipid zur Verwendung für die Behandlung einer oder mehrerer Fettstoffwechselerkrankungen, Fettleibigkeit, mit Fettstoffwechsel in Beziehung stehender Zustände oder Erkrankungen, oder mit Fettleibigkeit in Beziehung stehender Zustände oder Erkrankungen, wobei die Fettstoffwechselerkrankung aus einer oder mehreren von Hypertriglyceridämie, hoher Konzentration an freien Fettsäuren, und niedriger Konzentration an High-Density Lipoprotein-Cholesterin (Hypo-HDL-C) ausgewählt ist, und wobei gegebenenfalls die Fettstoffwechselerkrankung mit Fettleibigkeit assoziiert ist und die mit Fettstoffwechsel in Beziehung stehenden Zustände oder Erkrankungen und die mit Fettleibigkeit in Beziehung stehenden Zustände oder Erkrankungen aus mit Fettstoffwechsel oder Fettleibigkeit in Beziehung stehenden Organerkrankungen oder -fehlfunktionen, Proteinurie, Retinopathie, Neuropathie oder Nephropathie ausgewählt sind.

2. Nitriertes Lipid zur Verwendung nach Anspruch 1, wobei sich in einem Subjekt der Spiegel an einem oder mehreren von Triglyceriden, freien Fettsäuren, Körpergewicht, Cholesterin, Harnproteinausscheidung und oxidativem Stress verringert oder der Spiegel an Plasma-HDL erhöht, bezogen auf den Spiegel oder die Rate in dem Subjekt vor der Behandlung mit dem nitrierten Lipid.

3. Nitriertes Lipid zur Verwendung nach Anspruch 1, wobei das nitrierte Lipid an ein Subjekt zu verabreichen ist, dem weiterhin ein oder mehrere therapeutische Mittel verabreicht werden, wobei das therapeutische Mittel aus Mitteln ausgewählt ist, die für die Behandlung der in Anspruch 1 definierten Fettstoffwechselerkrankungen, von Fettleibigkeit, oder der in Anspruch 1 definierten, mit Fettstoffwechsel in Beziehung stehenden Zustände oder Erkrankungen oder mit Fettleibigkeit in Beziehung stehenden Zustände oder Erkrankungen von Nutzen sind.

4. Nitriertes Lipid zur Verwendung nach Anspruch 1, wobei das nitrierte Lipid eine einfach ungesättigte Fettsäure oder eine mehrfach ungesättigte Fettsäure umfasst.

5. Nitriertes Lipid zur Verwendung nach Anspruch 4, wobei die einfach ungesättigte Fettsäure Ölsäure ist.

6. Nitriertes Lipid zur Verwendung nach Anspruch 5, wobei die einfach ungesättigte Fettsäure aus 9-Nitroölsäure, 10-Nitroölsäure, oder Kombinationen hiervon ausgewählt ist.

7. Nitriertes Lipid zur Verwendung nach Anspruch 1, wobei das nitrierte Lipid in einer Menge im Bereich von etwa 1 µg bis 1 g pro Tag zu verabreichen ist.

8. Nitriertes Lipid zur Verwendung nach Anspruch 2, wobei sich das Körpergewicht des Subjekts, bezogen auf das Körpergewicht des Subjekts vor der Behandlung mit dem nitrierten Lipid, verringert.

9. Nitriertes Lipid zur Verwendung nach Anspruch 8, wobei die Verringerung 5% bis 15% beträgt.

10. Nitriertes Lipid zur Verwendung nach Anspruch 2, wobei sich der Spiegel an Triglyceriden in dem Subjekt, bezogen auf den Spiegel in dem Subjekt vor der Behandlung mit dem nitrierten Lipid, verringert.

11. Nitriertes Lipid zur Verwendung nach Anspruch 10, wobei die Verringerung 10% bis 50% beträgt.

12. Nitriertes Lipid zur Verwendung nach Anspruch 2, wobei sich der Spiegel an freien Fettsäuren in dem Subjekt, bezogen auf den Spiegel in dem Subjekt vor der Behandlung mit dem nitrierten Lipid, verringert, und wobei die Verringerung gegebenenfalls 10% bis 90% beträgt.

13. Nitriertes Lipid zur Verwendung nach Anspruch 2, wobei sich der Spiegel an Harnproteinausscheidung in dem Subjekt, bezogen auf den Spiegel in dem Subjekt vor der Behandlung mit dem nitrierten Lipid, verringert, und wobei sich die Rate der Harnproteinausscheidung gegebenenfalls um 20% bis 70% verringert.

14. Nitriertes Lipid zur Verwendung nach Anspruch 2, wobei sich der Spiegel an Plasma-HDL in dem Subjekt, bezogen auf den Spiegel in dem Subjekt vor der Behandlung mit dem nitrierten Lipid, erhöht, und wobei sich der Spiegel an Plasma-HDL gegebenenfalls um 10% bis 90% erhöht.

15. Nitriertes Lipid zur Verwendung nach Anspruch 1, wobei das Subjekt fettleibig oder pathologisch fettleibig ist.

## Revendications

1. Lipide nitré pour son utilisation dans le traitement d'un ou de plusieurs des cas suivants :
troubles lipidiques, obésité, affections ou troubles liés aux lipides, ou affections ou troubles liés à l'obésité, où le trouble lipidique est choisi parmi un ou plusieurs troubles de type hyper-triglycéridémie, concentration élevée d'acides gras libres, et basse concentration de lipoprotéines haute densité-cholestérol (hypo-HDL-C) et éventuellement où le trouble lipidique est associé à l'obésité et les affections ou troubles liés aux troubles et les affections ou troubles liés à l'obésité sont choisis parmi les lésions ou la dysfonction d'organes liés aux lipides ou liées à l'obésité, la protéinurie, la rétinopathie, la neuropathie, ou la néphropathie.

2. Lipide nitré pour son utilisation selon la revendication 1, où le niveau d'un ou plusieurs des triglycérides, des acides gras libres, du poids corporel, du cholestérol, de l'excrétion des protéines urinaires, du stress oxydatif est réduit ou le niveau des HDL plasmatiques est accru chez un sujet par rapport au niveau ou au taux chez ledit sujet avant le traitement avec le lipide nitré.

3. Lipide nitré pour son utilisation selon la revendication 1, où le lipide nitré doit être administré à un sujet auquel est également administré un ou plusieurs agents thérapeutiques, l'agent thérapeutique étant choisi parmi les agents utiles dans le traitement des troubles lipidiques définis dans la revendication 1, de l'obésité ou des affections ou troubles liés aux lipides ou des affections ou troubles liés à l'obésité définis dans la revendication 1.

4. Lipide nitré pour son utilisation selon la revendication 1, où le lipide nitré comprend un acide gras mono-insaturé ou un acide gras poly-insaturé.

5. Lipide nitré pour son utilisation selon la revendication 4, où l'acide gras mono-insaturé est l'acide oléique.

6. Lipide nitré pour son utilisation selon la revendication 5, où l'acide gras mono-insaturé est choisi parmi l'acide 9-nitro-oléique, l'acide 10-nitro-oléique, ou leurs combinaisons.

7. Lipide nitré pour son utilisation selon la revendication 1, où le lipide nitré doit être administré en une quantité allant d'environ 1 µg à 1 g par jour.

8. Lipide nitré pour son utilisation selon la revendication 2, où le poids corporel du sujet est réduit, par rapport au poids dudit sujet avant le traitement avec le lipide nitré.

9. Lipide nitré pour son utilisation selon la revendication 8, où la réduction est de 5 à 15 %.

10. Lipide nitré pour son utilisation selon la revendication 2, où le niveau des triglycérides chez le sujet est réduit, par rapport au niveau chez ledit sujet avant le traitement avec le lipide nitré.

11. Lipide nitré pour son utilisation selon la revendication 10, où la réduction est de 10 à 50 %.

12. Lipide nitré pour son utilisation selon la revendication 2, où le niveau d'acides gras libres chez le sujet est réduit, par rapport au niveau chez ledit sujet avant le traitement avec le lipide nitré, et éventuellement où la réduction est de 10 à 90 %.

13. Lipide nitré pour son utilisation selon la revendication 2, où le niveau d'excrétion des protéines urinaires chez le sujet est réduit, par rapport au niveau chez ledit sujet avant le traitement avec le lipide nitré, et éventuellement où le taux d'excrétion des protéines urinaires est réduit de 20 à 70 %.

14. Lipide nitré pour son utilisation selon la revendication 2, où le niveau des HDL plasmatiques chez le sujet est accru, par rapport au niveau chez ledit sujet avant le traitement avec le lipide nitré, et éventuellement où le niveau des HDL plasmatiques est accru de 10 à 90 %.

15. Lipide nitré pour son utilisation selon la revendication 1, où le sujet souffre d'obésité ou d'obésité morbide.
